# EUROPEAN PATENT SPECIFICATION

(11) **EP 1 613 277 B1**
(45) Date of publication and mention of the grant of the patent: **08.11.2006**
(21) Application number: 04719403.0
(22) Date of filing: 11.03.2004
(51) Int. Cl.: A61K 8/02

(54) **FRAGANCED SOLID COSMETIC COMPOSITIONS BASED ON A DESTRUCTURIZED STARCH DELIVERY SYSTEM**
PARFÜRMIERTE FESTE KOSMETISCHE ZUBEREITUNG BASIEREND AUF EINEM FREISETZUNGSSYSTEM AUS DESTRUKTURIERTER STÄRKE
COMPOSITIONS COSMETIQUES SOLIDES ET PARFUMEES REPOSANT SUR UN SYSTEME D'APPORT D'AMIDON DESTRUCTURE

(30) Priority: 10.04.2003 US 461675 P
(43) Date of publication of application: 11.01.2006
(73) Proprietor: UNILEVER PLC, London EC4P 4BQ (GB); UNILEVER N.V., 3013 AL Rotterdam (NL)
(72) Inventor: GOTT, Robert, Edward, 21614 Buxtehude (DE); SCHMITT, William, Howard Unilever Home and, Trumbull, Connecticut 06611 (US); SABIN, Robert, Daniel Unilever, Trumbull, Connecticut 06611 (US); LONDIN, Jonathan, Russell Unilever Home and, Trumbull, Connecticut 06611 (US); DOBKOWSKI, Brian, John Unilever Home and, Trumbull, Connecticut 06611 (US); CHENEY, Michael, Charles Unilever Home and, Trumbull, Connecticut 06611 (US); VINSKI, Paul Unilever Home and Personal Care USA, Trumbull, Connecticut 06611 (US); SLAVTCHEFF, Craig, Stephen Unilever Home and, Trumbull, Connecticut 06611 (US); PAREDES, Rosa, Mercedes, 40 Merritt Boulevard Trumbull, CT 06611 (US)
(74) Representative: Acham, Nicholas Clive
(86) International application number: PCT/EP2004/002560
(87) International publication number: WO 2004/089315

(56) References cited:
- EP-A- 1 160 311
- GB-A- 2 214 919
- US-A- 6 007 830
- US-A1- 2001 008 635
- US-A1- 2002 187 223
- US-A1- 2004 048 759
- DATABASE WPI Section Ch, Week 197701 Derwent Publications Ltd., London, GB; Class A96, AN 1977-01151Y XP002286362 & JP 51 133436 A (UOZUMI T) 19 November 1976 (1976-11-19)

## Description

### Field of the Invention

The invention is concerned with fragranced starch based solid cosmetic compositions, especially foamed solids such as pellets, releasing skin benefit ingredients upon contact of the solids with water.

### The Related Art

Many commercial cosmetic products contain water as the single largest formulation component. Other than its carrier function, water delivers but little cosmetic benefit. Removal of water results in concentrated products needing less packaging, less shelf space and incurs lower transportation costs. Any water necessary for the formulation may later be added by the eventual consumer during application of the product. Essentially waterless systems are therefore ecologically more friendly.

Beyond ecological considerations, there has been a trend towards the marketing of unit dose products. For instance, U.S. Patent 5,063,057 (Spellman et al.) and U.S. Patent 5,270,054 (Bertolini et al.) have disclosed gelatin capsules filled with anhydrous liquid skin treatments. These are released by puncture or through twisting off a neck segment of the capsule.

Biodegradable hydrophilic polyurethane foams have been reported in U.S. Patent 4,132,839 (Marans et al.). These foams are said to have utility as handy expandable sponges for personal use. Reportedly they may be prepared with detergents, lotions, perfumes and biostats.

In U.S. Patent 4,873,085 (Fuisz) a sugar in fiber form is described containing a cosmetic active agent releasable when the fibers are dissolved in water.

A body of literature has developed surrounding biodegradable starches. Many are based upon destructurized starches that can be foamed or molded into various shapes. Included in this literature are U.S. Patent 4,900,361 (Sachetto et al.), U.S. Patent 5,382,611 (Stepto et al.), U.S. Patent 5,480,923 (Schmid et al.), U.S. Patent 5,852,114 (Loomis et al.), U.S. Patent 6,001,385 (Van De Wijdeven) and U.S. Patent 6,277,899 B1 (Bastiolli et al.). Of particular interest for cosmetics are U.S. Patent 5,763,500, U.S. Patent 5,925,380 and U.S. Patent 6,007,830 all assigned to L'Oreal. These patents disclose expanded starch-rich thermoplastic hollow particles useful as make-up or hygiene compositions activated through re-hydration.

A problem with the known art is delivery of a fragrance with the cosmetic formulated starch-derived solids.

### SUMMARY OF THE INVENTION

A solid cosmetic composition, preferably a foamed solid cosmetic composition is provided including:
(i) a water dissolvable solid, preferably foamed, carrier comprising a destructurized starch;
(ii) at least one cosmetic agent incorporated into the solid carrier in an amount sufficient to provide a cosmetic benefit; and
(iii) a fragrance deposited as a post-dose onto the solid carrier that comprises destructurized starch and the at least one cosmetic agent.

### DETAILED DESCRIPTION OF THE INVENTION

Now it has been found that a fragrance is expressed with greater intensity when added to a destructurized starch rather than one incorporated into an unmodified type of starch.

The solid material is preferably formed into tablets, pellets, sheets, beads or shaped articles (e.g. a sculpted fish). When immersed in water, the solid and preferably foamed starch rapidly dissolves releasing the cosmetic agent which can then deposit or treat human skin and/or hair.

The term "destructurized starch" means that a starch is substantially released from the cells and granules in which it is naturally found. More particularly, "destructurized starch" is a molecular dispersion of starch and water. When formulated this material is molecularly homogeneous (with both amylose and amylopectin dispersed uniformly throughout the material), is amorphous, has no ordered molecular structure, has relatively high molecular-weight amylopectin, is not highly brittle or friable and has superior mechanical properties.

"Destructurized starch" is generated under certain conditions of temperature, pressure, shear, limited water and sufficient time. For instance, natural starch can be treated at elevated temperature in a closed vessel, thereby at elevated pressure, to form a melt. A typical process is conveniently carried out in an injection molding machine or extruder. The starch is fed through a hopper onto a rotating, reciprocating screw. The feed material moves along the screw towards the tip. During this process, temperature of the starch material is increased by means of external heaters around a barrel of the extruder and by the shearing action of the screw. The particulate feed becomes gradually molten starting in the feed zone and continuing in the compression zone. Molten feed is then conveyed through the metering zone, where homogenization of the melt occurs, to the end of the screw. Molten material at the tip can then be further treated by injection molding or extrusion or any other known technique to treat thermoplastic melts.

Starting natural starch material useful in the present invention preferably has a high amylose content (particularly is one of at least 45% by weight of amylose). It is well known that natural starch is composed of two fractions, the molecular arrangement of one being linear and the other being branched. The linear fraction of starch is known as amylose and the branched fraction amylopectin. Starches from different sources, e.g., potato, corn, tapioca and rice, etc., are characterized by different relative proportions of the amylose and amylopectin components. Some plant species have been genetically developed which are characterized by a large preponderance of one fraction over the other. For instance, certain varieties of corn which normally contain amylose have been developed which yield starch composed of over 45% amylose. These hybrid varieties have been referred to as high amylose or amylomaize.

Suitable high amylose starches useful herein are any starches with an amylose content of at least 45%, preferably at least 65% by weight. While high amylose corn starch has been especially suitable, other starches which are useful include those derived from any plant species which produces or can be made to produce a high amylose content starch, e.g., corn, peas, barley and rice. Additionally, high amylose starch can be obtained by separation or isolation such as the fractionation of a native starch material or by blending isolated amylose with a native starch.

In preparing the shaped products of this invention, an extrusion process, either alone or in combination with other forming operations, may be used depending on the desired type of final product. The expanded starch product leaving the extruder is typically in a rope or cylindrical form. By varying the size and configuration of the die opening of the extruder, different forms such as sheets of varying thickness and widths, irregular profiles and other shapes may be obtained. When expanded products of different shapes and design are desired, other forming operations subsequent to the extrusion operation may be utilized. One such readily adaptable technique involves thermoforming. In this operation, a material is heated to a temperature at which it is pliable or shapable and then forced against a mold by applying vacuum, air or mechanical pressure. After the expanded starch product of this invention leaves the extruder, it is still quite hot and malleable and therefore well suited for the thermoforming step.

Other methods of forming the solid cosmetic products, particularly the foamed solids, may be used in addition to the extrusion/thermoforming operations discussed above. Such methods include injection molding, blow molding, extrusion-blow molding and stamping, as well as combinations of these and other methods.

A particularly advantageous form of the foamed solid cosmetic products of this invention is that of a honeycomb. By the term honeycomb is meant a round or polygonal shaped object from which smaller pieces may be broken off, the smaller pieces being defined by fracture or fracturable sections of the honeycomb. When a user requires the product, the user may break off a section from the honeycomb and throw this into their bath.

Solid carriers and particularly foamed solid carriers of this invention advantageously are but not limited to being expanded, biodegradable starch material having a uniform, closed cell structure with low density and good resilience and compressibility properties. The uniform, closed cell structure with its characteristic tiny bubble formation, not only results in a Styrofoam-like appearance and density, but gives it resilience and compressibility. A closed cell structure is defined as one having largely nonconnecting cells, as opposed to open cells which are largely interconnecting or defined as two or more cells interconnected by broken, punctured or missing cell walls. The tiny bubble formation generally results in a small cell size of typically about 100 to 600 microns. This is accomplished by extrusion of a high amylose starch at a total moisture or water content of 21% or less by weight and at a temperature of from about 150° to 250°C.

Amount of destructurized starch within the solid carrier may range from about 10 to 100%, preferably from 50 to 100%, and optimally about 100%. Modified starch may also be used in forming the foamed solid carrier. The amount of total foamed solid carrier may range from about 10% to about 99%, preferably from about 70 to about 95% by weight of the composition. By "modified" is meant that the starch can be derivatized through a typical chemical reaction such as esterification, etherification, oxidation, acid hydrolysis, cross-linking and enzyme conversion. Typically, modified starches include esters, such as acetates and the half-esters of dicarboxylic acids, particularly the alkenylsuccinate acids; ethers, such as the hydroxyethyl- and hydroxypropyl-starches and starches reacted with hydrophobic cationic epoxides; starches oxidized with hypochlorite; starches reacted with cross-linking agents such as phosphorous oxychloride, epichlorohydrin, and phosphate derivatives prepared by reaction with sodium or potassium orthophosphate or tripolyphosphate and combinations thereof. These and other conventional modifications of starch are described in publications such as "Starch: Chemistry and Technology", Second Edition, edited by Roy L. Whistler et al. Chapter X: Starch Derivatives: Production and Uses by M.W. Rutenberg et al., Academic Press, Inc., 1984.

One especially advantageous modification of the starches that may be used in this invention is the etherification with alkylene oxides, particularly those containing 2 to 6, preferably 2 to 4, carbon atoms. Ethylene oxide, propylene oxide and butylene oxide are exemplary compounds useful in etherifying the starting starch materials, with propylene oxide being especially preferred. Varying amounts of such compounds may be used depending on the desired properties and economics. Generally, up to 15% or more and preferably, up to about 10% by weight, based on the weight of starch will be used.

Solid carriers and particularly solid foamed carriers of the present invention may be based upon a mixture of destructurized starch and a synthetic plastic in ratios which may range from 10:1 to 1:10. The synthetic plastic for illustrative purposes only may be selected from one or more of the following:
(A) Polyvinyl alcohol, polyvinyl acetate and copolymers of an olefin selected from ethylene, propylene, isobutene and styrene with one or more monomers selected from acrylic acid, methacrylic acid, C₁-C₄ alkylacrylate, C₁-C₄ alkylmethacrylate, vinyl alcohol, vinyl acetate and maleic acid, such as ethylene-acrylic acid, ethylene-vinyl alcohol, ethylene-vinyl acetate, ethylene-maleic anhydride copolymers and their mixtures; the particularly preferred compounds are ethylene-vinyl alcohol copolymers with an ethylene content of from 10-44% by weight produced by the hydrolysis of the corresponding poly-ethylene-vinyl acetate with a degree of hydrolysis of between 50 and 100;
(B) Thermoplastic polyesters such as, in particular, homopolymers and copolymers of hydroxyaliphatic acids having from 2 to 24 carbon atoms, preferably 2 to 8 carbon atoms, the corresponding lactones or lactides and polyesters derived from bifunctional carboxylic acids with aliphatic diols.
   In particular, in group B the preferred thermoplastic and mixtures of thermplastics selected are:
B1) poly-epsilon-caprolactone, copolymers of epsilon-caprolactone with isocyanates such as, in particular, 4,4'-diphenylmethane diisocyanate, toluylene diisocyanate, isophorone diisocyanate or hexamethylene diisocyanate;
B2) polymers of lactic acid or polylactides, of glycolic acid or polyglycolides, copolymers of lactic acid and glycolic acid;
B3) polyhydroxybutyrate, polyhydroxybutyrate/valerate;
B4) polymers derived from dicarboxylic acids with aliphatic diols such as, in particular, polyethylene and polybutylene adipate or sebacate;
B5) block or graft copolymers formed between homopolymers and copolymers of hydroxyacids, in particular poly-epsilon-caprolactone, and one or more of the following components:
   i) cellulose or modified cellulose, for example, carboxymethylcellulose and cellulose acetate;
   ii) amylose, amylopectin, natural or modified starches;
   iii) polymers resulting from the reaction of a compound selected from aliphatic diols (such as ethylene glycol, propylene glycol, butylene glycol, polyoxyethylene glycol, polyoxypropylene glycol, neopentyl glycol, 1,4-butanediol, cyclohexanediol), prepolymers or polymers of polyesters having terminal diol groups with monomers selected from: bifunctional aromatic or aliphatic isocyanates, bifunctional aromatic or aliphatic epoxides, dicarboxylic aliphatic acids (such as malonic, succinic, maleic, fumaric, itaconic, glutaric, adipic, pimelic, suberic, azaleic and sebacic acids), dicarboxylic cycloaliphatic acids (such as cyclohexanedicarboxylic acid or 2,2,3-dicyclooctanedicarboxylic acid) or aromatic acids or anhydrides (such as phthalic acid),
   iv) polyurethanes, polyamides-urethanes from diisocyanates and aminoalcohols, polyamides, polyesters-amides from dicarboxylic acids and aminoalcohols, polyesterurea from aminoacids and diesters of glycols,
   v) polyhydroxy polymers (such as polyvinyl alcohol, ethylene-vinyl alcohol copolymers, totally or partially hydrolysed),
   vi) polyvinyl pyrrolidone, polyvinyl pyrrolidone-vinyl-acetate copolymers and polyethyloxazoline,
B6) polyesters obtained from monomers of hydroxyacids upgraded with chain lengtheners such as isocyanates, epoxides, phenylesters and aliphatic carbonates;
B7) polyesters obtained from monomers of hydroxyacids partly cross-linked with polyfunctional acids such as trimellitic acid, pyromellitic acid, polyisocyanates and polyepoxides.
(C) Graft copolymers of polysaccharides and their derivatives, such as starches, cellulose, modified cellulose, rubbers, alginates, pectins, dextrins and pullulans with monomers such as styrene, methylmethyacrylate, methylacrylate, butylacrylate, butadiene, isoprene, acrylonitrile, graft copolymers of polysaccharides are described in the Encyclopedia of Polymer Science and Engineering, John Wiley & Sons, Volume 3, 1986.

Most preferred for purposes of this invention are foamed solid carriers being comprised substantially all of destructurized starch. Most preferred commercial source of destructurized starch are the materials known as Eco-Maize^{®}, Eco-Tapioca^{®}, Eco-Plus^{®}, Eco-Foam^{®} and Eco-Shape 47^{®}, all available from the National Starch & Chemical Company.

The term "fragrance" is defined as a mixture of odoriferous components, optionally mixed with a suitable solvent diluent or carrier, which is employed to impart a desired odor.

Fragrance components and mixtures thereof may be obtained from natural products such as essential oils, absolutes, resinoids, resins and concretes, as well as synthetic products such as hydrocarbons, alcohols, aldehydes, ketones, ethers, carboxylic acids, esters, acetals, ketals, nitriles and the like, including saturated and unsaturated compounds, aliphatic, carbocyclic and heterocyclic compounds.

Suitable characteristics of such perfumes/fragrances can include at least one of the following, in any combination: (1) liquid or semi-liquid after mixing with the other components; (2) pleasant and/or clean odor when mixed with other components, e.g., one or more of lavender, violet, rose, jasmin, pine, woody, floral, fruity, lemon, lime, apple, peach, raspberry, strawberry, banana, plum, apricot, vanilla, pear, eucalyptus, aromatic, aldehydic, tutti frutti, oriental, sweet, amber, Paola, Muguet, Citron (lime) ella, and the like; (3) specific gravity (20°C/20°C) in the range of 0.600-1.300, preferably 0.800-1.100, each preferably varying 0.001-0.05, more preferably 0.008-0.020; (4) refractive index (20°C) of 1.300-1.800, preferably 1.400-1.600, each preferably varying 0.001-0.05, more preferably 0.008-0.020; (5) saponification value of 5-300, preferably 10-250; and (6) having a flash point of 20-200 Pensky-Martens Closed Cup (P.M.C.C.) and 10-100 Tag-Closed Cup (T.C.C.).

Typical fragrance ingredients which may be employed for the present invention can be selected from one or more of:
2-methoxy naphthalene
Allyl cyclohexane propionate
alpha-citronellal
alpha-ionone
alpha-Santalol
alpha-Terpineol
Ambrettolide
Amyl benzoate
Amyl cinnamate
Amyl cinnamic aldehyde
Aurantiol
Benzaldehyde
Benzophenone
Benzyl acetate
Benzyl salicylate
Beta-caryophyllene
beta-Methyl naphthyl ketone
Cadinene
Cavacrol
Cedrol
Cedryl acetate
Cedryl formate
Cinnamyl cinnamate
cis-Jasmone
Coumarin
Cyclamen aldehyde
Cyclohexyl salicylate
d-Limonene
delta-Nonalactone
delta-Undecalactone
Dihydro isojasmonate
Dihydro mycenol
Dimethyl acetal
Diphenyl methane
Diphenyl oxide
Dodecalactone
Ethyl methyl phenyl glycidate
Ethyl undecylenate
Ethylene brassylate
Eugenol
Exaltolide
Galaxolide
gamma-n-methyl ionone
gamma-Undecalactone
Geranial
Geranyl acetate
Geranyl anthranilate
Geranyl phenyl acetate
Hexadecanolide
Hexenyl salicylate
Hexyl cinnamic aldehyde
Hexyl salicylate
Hydroxycitronellal
Indole
Iso E super
Iso-Amyl salicylate
Iso-bornyl acetate
Iso-butyl quinoline
Iso-Eugenol
Laevo-Carvone
Lilial (p-t-bucinal)
Linalool
Linalyl acetate
Linalyl benzoate
Methyl cinnamate
Methyl dihydrojasmonate
Methyl-N-methyl anthranilate
Musk indanone
Musk ketone
Musk tibetine
Myristicin
Nerol
Oxahexadecanolide-10
Oxahexadecanolide-11
para-cymene
para-tert-Butyl cyclohexyl acetate
Patchouli alcohol
Phantolide
Phenyl ethyl alcohol
Phenyl ethyl benzoate
Phenyl heptanol
Phenylhexanol
Phenylethylphenylacetate
Thibetolide
Vanillin
Vertenex
Vetiveryl acetate
Yara-yara
Ylangene

Suitable solvents, diluents or carriers for fragrance components as mentioned above are for example: ethanol, isopropanol, diethylene glycol monoethyl ether, dipropyl glycol, triethyl citrate and the like.

Particularly preferred fragrance components of the present invention are cyclic and acyclic terpenes and terpenoids. These materials are based upon isoprene repeating units. Examples include alpha and beta pinene, myrcene, geranyl alcohol and acetate, camphene, dl-limonene, alpha and beta phellandrene, tricyclene, terpinolene, allocimmane, geraniol, nerol, linanool, dihydrolinanool, citral, ionone, methyl ionone, citronellol, citronellal, alpha terpineol, beta terpineol, alpha fenchol, borneol, isoborneol, camphor, terpinen-1-ol, terpin-4-ol, dihydroterpineol, methyl chavicol, anethole, 1,4 and 1,8 cineole, geranyl nitrile, isobornyl acetate, linalyl acetate, caryophyllene, alpha cedrene, guaiol, patchouli alcohol, alpha and beta santalol and mixtures thereof.

Fragrance is applied to the solid carrier (preferably foamed solid carrier) in a variety of ways. Particularly preferred is dosage through a spray application onto the destructurized starch solids. Alternatively, the fragrance can be deposited by gravity feed in the form of droplets deposited onto the solid carrier.

Amounts of fragrance may range from 0.001 to 10%, preferably from 0.1 to 5%, optimally from 0.5 to 3% by weight of the solid cosmetic composition.

The bulk density of the composition may but will not necessarily be limited to from 0.1 to 5 lb/ft³ (0. 0016 to 0.08 g/cm³), and preferably from 0.2 to 3.09 lb/ft³ (0.0032 to 0.0495 g/cm³). Compressibility will range from 100 to 800, preferably from 150 to 700 and more preferably from 400 to 600 g/cm².

A second essential component of compositions according to this invention is a cosmetic agent. The cosmetic agent may be present at levels effective to provide a benefit to skin and/or hair to which it is applied. Generally, the amount of the agent will range anywhere from 0.001 to 50%, preferably from 0.1 to 30%, optimally from 1 to 15% by weight. The relative weight ratio of foamed solid carrier to cosmetic agent may range from 1000:1 to 1:1, preferably from 20:1 to 5:1.

Typical cosmetic agents may include surfactants, emollients, humectants, conditioners, sunscreens, anti-aging actives and combinations thereof.

Illustrative surfactants are those selected from anionic, nonionic, cationic and amphoteric actives. Particularly preferred nonionic surfactants are those with a C₁₀-C₂₀ fatty alcohol or acid hydrophobe condensed with from about 2 to about 100 moles of ethylene oxide or propylene oxide per mole of hydrophobe, C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amines (e.g. methyl gluconamides) are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionates, C₈-C₂₀ alkyl ether phosphates and combinations thereof.

Amphoteric surfactants include such materials as dialkyl amine oxide and various types of betaines such as cocoamido propyl betaine.
Surfactants can comprise anywhere from 0.5 to 50%, preferably from 1 to 30%, optimally from 5 to 15% by weight of the total composition.

Illustrative emollients for use as the cosmetic agent in the present invention are materials such as esters, silicone oils, fatty acids and alcohols, hydrocarbons and mixtures thereof.

Representative esters suitable for the present invention are:
(1) Alkyl esters of fatty acids having 10 to 20 carbon atoms. Methyl, isopropyl, and butyl esters of fatty acids are useful herein. Examples include hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate. Particularly preferred are C₁₂-C₁₅ alcohol benzoate esters.
(2) Alkenyl esters of fatty acids having 10 to 30 carbon atoms. Examples thereof include oleyl myristate, oleyl stearate and oleyl oleate.
(3) Ether-esters such as fatty acid esters of ethoxylated fatty alcohols.
(4) Polyhydric alcohol esters, ethylene glycol mono and di-fatty acid esters, diethylene glycol mono- and di- fatty acid esters, polyethylene glycol (200-6000) mono- and di-fatty acid esters, propylene glycol mono- and di- fatty acid esters, polypropylene glycol 2000 monooleate, polypropylene glycol 2000 monstearate, ethoxylated propylene glycol monostearate, glyceryl mono- and di- fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(5) Wax esters such as beeswax, spermaceti, myristyl myristateand stearyl stearate.
(6) Sterols esters, of which cholesterol fatty acid esters are examples thereof.

Silicone oils may also be used as emollients. These oils may be either volatile or nonvolatile. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms.

Linear volatile silicone materials generally have viscosities less than 5 mm²/s (centistokes) at 25°C while cyclic materials typically have viscosities of less than 10 mm²/s (centistokes).

Examples of preferred volatile silicone oils useful herein include: Dow Corning 344, Dow Corning 345 and Dow Corning 200 (manufactured by Dow Corning Corp.); Silicone 7207 and Silicone 7158 (manufactured by the Union Carbide Corp.); SF 1202 (manufactured by General Electric); and SWS-03314 (manufactured by SWS Silicones, Inc.).

The nonvolatile silicone oils useful in compositions of this invention are exemplified by the polyalkyl siloxanes, polyalkyaryl siloxanes and polyether siloxane copolymers. The essentially nonvolatile polyalkyl siloxanes useful herein include, for example, polydimethyl siloxanes with viscosities of from about 5 to about 100,000 mm²/s (centistokes) at 25°C. Such polyalkyl siloxanes include the Viscasil series (sold by General Electric Company) and the Dow Corning 200 series (sold by Dow Corning Corporation). Polyalkylaryl siloxanes include poly (methylphenyl) siloxanes having viscosities of from about 15 to about 65 mm²/s (centistokes) at 25°C. These are available, for example, as SF 1075 methylphenyl fluid (sold by General Electric Company) and 556 Cosmetic Grade Fluid (sold by Dow Corning Corporation). Useful polyether siloxane copolymers include, for example, a polyoxyalkylene ether copolymer having a viscosity of about 1200 to 1500 mm²/s (centistokes) at 25°C. Such a fluid is available as SF-1066 organosilicone surfactant (sold by General Electric Company). Cetyl dimethicone copolyol and cetyl dimethicone are especially preferred because these materials also function as emulsifiers and emollients.

In certain types of compositions according to the present invention, the amount of silicone may need to be highly regulated. Besides their emollient properties, silicones can have a significant effect upon foaming properties. For instance, when the compositions of the present invention are intended to be hydratable into a shaving cream, a controlled amount of silicone may be necessary as an anti-foam to quench any foaming by surfactants in the shaving cream which undesirably may tend to foam during the manufacturing process. On the other hand, too much silicone may prevent eventual foaming when a product such as a shaving cream or shampoo (when hydrated) is ready for utilization by a consumer. Amounts of the silicone for anti-foam purposes may range from 0.00001 to 1%, preferably from 0.0001 to 0.01, optimally from 0.05 to 0.1% by weight of the composition.

Suitable fatty alcohols and acids include those compounds having from 10 to 24 carbon atoms. Especially preferred are such compounds as cetyl, myristyl, palmitic and stearyl alcohols and acids.

Exemplary hydrocarbons which may serve as emollients are those having hydrocarbon chains anywhere from 12 to 30 carbon atoms. Specific examples include mineral oil, petroleum jelly, squalene and isoparaffins.

Amounts of the emollients may range from 0.1 to 99%, preferably from 25 to 90%, optimally from 35 to 75% by weight of the composition.

Humectants of the polyhydric alcohol-type may also be included in the compositions of this invention. The humectant aids in increasing the effectiveness of the emollient, reduces scaling, stimulates removal of built-up scale and improves skin feel. Typical polyhydric alcohols include glycerol, diglycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,2-butylene glycol, 1,2,6-hexanetriol, ethoxylated glycerol, propoxylated glycerol and mixtures thereof. For best results the humectant is preferably glycerol. The amount of humectant may range anywhere from 0.5 to 30%, preferably between 1 and 15% by weight of the composition.

Illustrative conditioners of the present invention are the quaternary ammonium salts. Typical of such materials are guar hydroxypropyltrimonium chloride and quaternized cellulose such as Polymer JR7.

Cosmetic agents of this invention may include sunscreens. These are materials commonly employed to block ultraviolet light. Suitable sunscreens are the derivatives of PABA, cinnamate and salicylate. For example, octyl methoxycinnamate and 2-hydroxy-4-methoxy benzophenone (also known as Oxybenzone) can be used. Octyl methoxycinnamate and 2-hydroxy-4-methoxy benzophenone are commercially available under the trademarks, Parsol MCX and Benzophenone-3, respectively. Also useful is AVOBENZENE sold as Parsol 1789. The exact amount of sunscreen employed can vary depending upon the degree of protection desired from the sun's UV radiation.

Anti-aging actives are best exemplified by the 2-hydroxyalkanoic acids, prostaglandins, retinoic acids, retinol and fatty esters thereof, ceramides and their derivatives. These agents may be present anywhere from 0.00001 to 15%, preferably from 0.0001 to 5%, optimally between 0.01 and 1% by weight of the total composition. Most preferred of the actives mentioned above are 2-hydroxyoctanoic acid, glycolic acid, lactic acid, retinol and pigskin or bovine-brain lipid ceramides as well as salts of these actives.

Vitamins may also be included as anti-aging actives. Especially preferred is vitamin A palmitate (retinyl palmitate), Vitamin C tetraisopalmitate (ascorbyl tetraisopalmitate) and vitamin E linoleate (tocopheryl linoleate). Other esters or alcohols of vitamins A and E may also be utilized.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

All documents referred to herein, including all patents, patent applications, and printed publications, are hereby incorporated by reference in their entirety in this disclosure.

The following examples will more fully illustrate selected embodiments of this invention. All parts, percentages and proportions referred to herein and in the appended claims are by weight unless otherwise indicated.

### EXAMPLE 1

Foamed solid cosmetic compositions are prepared by warming fatty or oil-based materials to 70°C to achieve fluidity. Other liquid or solid non-meltable materials are then dispersed into the resultant mass with thorough mixing. The product resulting therefrom is then added with mixing to a high amylose destructurized corn starch. This formed mass is then extruded at a temperature of 150-250°C. The extruded mass is then shaped. Fragrance is sprayed onto the shaped mass. In this manner a shampoo solid is prepared whose formula is listed below.

**SHAMPOO SOLID**

| INGREDIENTS | WEIGHT % |
|---|---|
| High Amylose Corn Starch | 86.39 |
| Sodium Lauryl Sulphate (Flakes) | 10.00 |
| Coconut Diethanolamide | 3.00 |
| Germall 115 | 0.20 |
| Fragrance* | 0.40 |
| Colorant | 0.01 |

| | |
|---|---|
| * Post-dosed on solid | |

### EXAMPLE 2

A conditioning shampoo solid whose formula is described below is prepared according to the method of Example 1.

**CONDITIONING SHAMPOO SOLID**

| INGREDIENT | WEIGHT % |
|---|---|
| High Amylose Corn Starch | 81.09 |
| Sodium Lauryl Sulphate (Flakes) | 10.00 |
| Cocamidopropyl Betaine (30% active) | 5.00 |
| Coconut Diethanolamide | 2.50 |
| Dimethicone Copolyol | 0.80 |
| Methyl Paraben | 0.10 |
| Sorbic Acid | 0.10 |
| Fragrance* | 0.40 |
| Colorant | 0.01 |

| | |
|---|---|
| * Post-dosed onto solid | |

### EXAMPLE 3

A conditioner solid whose formula is described below is prepared according to the method of Example 1.

**CONDITIONER SOLID**

| INGREDIENT | WEIGHT % |
|---|---|
| High Amylose Corn Starch | 83.59 |
| Dimethyl Dihydrogenated Tallow Ammonium Chloride 75% | 4.00 |
| Stearamidoethyl Diethylamine | 4.00 |
| Cetyl Alcohol | 4.00 |
| Methyl Paraben | 0.10 |
| Sorbic Acid | 0.10 |
| Citric Acid | 4.00 |
| Fragrance* | 0.20 |
| Colorant | 0.01 |

| | |
|---|---|
| * Post-dosed onto solid | |

### EXAMPLE 4

A setting/styling product solid whose formula is described below is prepared according to the method of Example 1.

**SETTING/STYLING PRODUCT SOLID**

| INGREDIENT | WEIGHT % |
|---|---|
| High Amylose Corn Starch | 91.39 |
| Polyvinyl Pyrrolidone K-30 | 8.00 |
| Cetyl Trimethyl Ammonium Chloride | 0.03 |
| Methyl Paraben | 0.10 |
| Sorbic Acid | 0.10 |
| Fragrance* | 0.10 |
| Colorant | 0.01 |

| | |
|---|---|
| * Post-dosed onto solid | |

### EXAMPLE 5

A hair cream solid whose formula is described below is prepared according to the method of Example 1.

**HAIR CREAM SOLID**

| INGREDIENT | WEIGHT % |
|---|---|
| High Amylose Corn Starch | 88.84 |
| Mineral Oil | 5.00 |
| Lanolin | 1.00 |
| Stearic Acid | 2.00 |
| Myristyl Myristate | 1.00 |
| Dimethicone | 1.00 |
| Triethanolamine | 0.75 |
| Methyl Paraben | 0.10 |
| Sorbic Acid | 0.10 |
| Fragrance* | 0.20 |
| Colorant | 0.01 |

| | |
|---|---|
| * Post-dosed onto solid | |

### EXAMPLE 6

A cleansing cream solid whose formula is described below is prepared according to the method of Example 1.

**CLEANSING CREAM SOLID**

| INGREDIENT | WEIGHT % |
|---|---|
| High Amylose Corn Starch | 88.60 |
| Sodium Cocoyl Isethionate | 6.00 |
| Cocamide MEA | 0.75 |
| POE-20 Sorbitan Monostearate | 0.50 |
| Ceteareth-20 | 0.75 |
| Cetyl Alcohol | 0.50 |
| Ethylene Glycol Monostearate | 0.30 |
| Isopropyl Palmitate | 2.00 |
| PEG-7 Glyceryl Cocoate | 0.25 |
| Methyl Paraben | 0.10 |
| Propyl Paraben | 0.05 |
| Fragrance* | 0.20 |

| | |
|---|---|
| * Post-dosed onto solid | |

### EXAMPLE 7

A hand lotion solid whose formula is described below is prepared according to the method of Example 1.

**HAND LOTION SOLID**

| INGREDIENT | WEIGHT % |
|---|---|
| High Amylose Corn Starch | 91.79 |
| Stearic Acid | 1.25 |
| Glyceryl Monostearate | 0.50 |
| Cetyl Alcohol | 0.50 |
| Petrolatum | 0.50 |
| Mineral Oil (70 vis) | 1.00 |
| Isopropyl Palmitate | 1.00 |
| PEG 40 Stearate | 0.10 |
| Glycerin | 2.50 |
| Triethanolamine | 0.50 |
| Methyl Paraben | 0.10 |
| Propyl Paraben | 0.05 |
| Fragrance* | 0:20 |
| Colorant | 0.01 |

| | |
|---|---|
| * Post-dosed onto solid | |

### EXAMPLE 8

A facial moisturizer solid whose formula is described below is prepared according to the method of Example 1.

**FACIAL MOISTURIZER SOLID**

| INGREDIENT | WEIGHT % |
|---|---|
| High Amylose Corn Starch | 89.17 |
| Glycerin | 1.00 |
| Propylene Glycol | 1.50 |
| Glyceryl Monostearate | 2.00 |
| Mineral Oil | 1.50 |
| Caprylic/Capric Triglycerides | 2.00 |
| Hydrogenated Safflower Oil | 0.75 |
| Stearic Acid | 1.00 |
| Laneth 23 | 0.40 |
| Triethanolamine | 0.30 |
| Vitamin A Palmitate | 0.10 |
| Vitamin E Linoleate | 0.10 |
| Methyl Paraben | 0.05 |
| Propyl Paraben | 0.01 |
| Fragrance* | 0.01 |
| Colorant | 0.01 |

| | |
|---|---|
| * Post-dosed onto solid | |

### EXAMPLE 9

A suntan lotion shampoo solid whose formula is described below is prepared according to the method of Example 1.

**SUNTAN LOTION SOLID**

| INGREDIENT | WEIGHT % |
|---|---|
| High Amylose Corn Starch | 90.54 |
| Ethylhexyl p-methoxycinnamate | 1.00 |
| Oxybenzone | 0.50 |
| Isopropyl Palmitate | 3.00 |
| Stearic Acid | 1.50 |
| Cetyl Alcohol | 0.50 |
| Glyceryl Monostearate | 0.50 |
| Sorbitol (70% soln.) | 1.50 |
| Triethanolamine | 0.60 |
| Methyl Paraben | 0.10 |
| Propyl Paraben | 0.05 |
| Fragrance* | 0.20 |
| Colorant | 0.01 |

| | |
|---|---|
| * Post-dosed onto solid | |

### EXAMPLE 10

A make-up or foundation whose formula is described below is prepared according to the method of Example 1.

**MAKE-UP OR FOUNDATION**

| INGREDIENT | WEIGHT % |
|---|---|
| High Amylose Corn Starch | 76.425 |
| Steareth-7/Stearyl Alcohol-Steareth 10 | 4.500 |
| Isooctadecyl Isononanoate | 1.000 |
| Isopropyl Myristate | 4.500 |
| Mineral Oil | 4.000 |
| Stearyl Dimethicone | 1.250 |
| Dimethicone | 0.250 |
| Glycerin | 1.000 |
| Talc | 2.000 |
| Titanium Dioxide | 3.000 |
| Iron Oxides | 1.750 |
| Ultramarine Blue | 0.075 |
| Methyl Paraben | 0.100 |
| Propyl Paraben | 0.050 |
| Fragrance* | 0.100 |

| | |
|---|---|
| * Post-dosed onto solid | |

### EXAMPLE 11

A baby cleanser solid whose formula is described below is prepared according to the method of Example 1.

**BABY CLEANSER SOLID**

| INGREDIENT | WEIGHT % |
|---|---|
| High Amylose Corn Starch | 90.58 |
| Mineral Oil (70 vis) | 6.00 |
| Dimethicone | 1.00 |
| Isopropyl Palmitate | 0.75 |
| POE Sorbitan Monooleate | 0.60 |
| Sorbitan Monooleate | 0.35 |
| Propylene Glycol | 0.50 |
| Methyl Paraben | 0.10 |
| Propyl Paraben | 0.01 |
| Fragrance* | 0.10 |
| Colorant | 0.01 |

| | |
|---|---|
| * Post-dosed onto solid | |

### EXAMPLE 12

A foam bath solid whose formula is described below is prepared according to the method of Example 1.

**FOAM BATH SOLID**

| INGREDIENT | WEIGHT % |
|---|---|
| High Amylose Corn Starch | 82.00 |
| Sodium Lauryl Sulfate (Flakes) | 15.00 |
| Coconut Diethanolamide | 1.50 |
| Methyl Paraben | 0.10 |
| Propyl Paraben | 0.10 |
| Fragrance* | 1.00 |
| Colorant | 0.30 |

| | |
|---|---|
| * Post-dosed onto solid | |

### EXAMPLE 13

A soluble bath oil solid whose formula is described below is prepared according to the method of Example 1.

**SOLUBLE BATH OIL SOLID**

| INGREDIENT | WEIGHT % |
|---|---|
| High Amylose Corn Starch | 84.39 |
| Polysorbate 20 | 5.00 |
| Polysorbate 80 | 5.00 |
| Fragrance* | 5.00 |
| Methyl Paraben | 0.10 |
| Propyl Paraben | 0.01 |
| Colorant | 0.50 |

| | |
|---|---|
| * Post-dosed onto solid | |

### EXAMPLE 14

A dispersible bath oil whose formula is described below is prepared according to the method of Example 1.

**DISPERSIBLE BATH OIL**

| INGREDIENT | WEIGHT % |
|---|---|
| High Amylose corn Starch | 74.19 |
| Mineral Oil (70 vis) | 10.00 |
| Corn Oil | 5.00 |
| Isopropyl Myristate | 5.00 |
| Oleth-3 | 5.00 |
| Fragrance* | 0.50 |
| Methyl Paraben | 0.10 |
| Propyl Paraben | 0.01 |
| Colorant | 0.20 |

| | |
|---|---|
| * Post-dosed onto product | |

### EXAMPLE 15

A series of re-hydratable lotions containing a variety of skin benefit agents are reported below. These are formulated with destructurized starch into foamed cosmetic compositions.

| | | | | | |
|---|---|---|---|---|---|
| | | | | | |

| Ingredient | A | B | C | D | E |
|---|---|---|---|---|---|
| Phase A | | | | | |
| Stearic Acid | 50.33 | 32.87 | 37.08 | | |
| Distearyl Dimonium Chloride | | | | 43.35 | 43.35 |
| Steareth 21 | | | | 17.27 | 17.27 |
| Glyceryl Monostearate/Stearamide AMP | 19.50 | 17.50 | 14.55 | | |
| Glyceryl Monostearate | 9.11 | 8.14 | 6.79 | | |
| Cetyl Alcohol | 5.20 | 4.67 | 3.89 | 27.26 | 27.26 |
| Sunflowerseed Oil | | 7.30 | | 12.12 | |
| Dimethicone | | 4.00 | 3.52 | | 12.12 |
| Petrolatum | | 15.20 | 12.51 | | |
| Mineral Oil | | | 16.22 | | |

| Phase B | | | | | |
|---|---|---|---|---|---|
| Potassium Hydroxide (45%) | | | 5.44 | | |
| Triethanolamine | 15.86 | 10.32 | | | |

A reactor is charged with the components listed under Phase A. The combination is melted at 85°C with strong mixing. Phase B is added to the reactor while maintaining a strong agitation. After stirring for 10 minutes, the combined phases are allowed to cool under moderate agitation. After the solution is cooled and solidified, the material is powderized within a blender, Hammermill or other suitable pulverization mechanism.

The foamed solids incorporating the re-hydratable lotions identified above are prepared according to the following process. An amount of the re-hydratable lotion sample is fed into a heated extruder along with zea mays (destructurized) starch at a temperature from 80 to 210°C. Relative weight ratio of lotion sample to starch is 10:90. In a first stage of this process the components are conveyed and mixed in an extruder for periods of time of the order of 2 to 50 seconds. During mixing, the blend is subjected to shearing stresses. Thereafter, a degassing phase occurs under vacuum to obtain a melt temperature from 130 to 180°C with a water content less than 5% to thereby avoid creation of bubbles at the outlet of the extruder. The melt is then extruded directly into stranded, spaghetti-like form. The strands are then fed between a set of rollers to form a sheet-like material. Fragrance oil at 0.10% by weight of the sheet is sprayed onto the sheet. Subsequent thereto, the sheet is cut into 10 x 20 cm towelettes. Each is packaged within a sealed foil pouch. When ready to use, a consumer removes the foil, places the towelette under water and thereby re-hydrates the lotion trapped within the starch carrier.

### EXAMPLE 16

A further series of skin lotions, in this instance without emulsifiers, are presented as formulations F through K.

| | Sample (wt. %) | | | | | |
|---|---|---|---|---|---|---|
| Ingredient | F | G | H | I | J | K |
| Dimethicone | 100.00 | | 50.00 | 25.00 | 15.00 | 40.00 |
| Dimethicone Copolyol | | | | | | 20.00 |
| Polysorbate 20 | | | | | 40.00 | |
| Dimethicone/Divinylmethicone Crosspolymer | | 100.00 | 50.00 | 25.00 | 15.00 | 40.00 |
| Petrolatum G1937 | | | | 25.00 | 15.00 | |
| Polyethylene | | | | 25.00 | 15.00 | |

The re-hydratable lotions after preparation are heated at 60°C under agitation. The mixtures are then each added to an extruder with zea mays (destructurized) starch at a 10% loading of lotion based on total solid composition. Sheets of foamed starch impregnated re-hydratable lotions are obtained from the extruder. The sheets are then sprayed with a fragrance oil achieving a loading of 0.1% by weight of the total foamed sheet.

### EXAMPLE 17

A set of re-hydratable self-heating lotions are formulated below intended for incorporation into foamed solid cosmetic compositions of the present invention.

| | Sample (wt. %) | | | |
|---|---|---|---|---|
| Description | L | M | N | O |
| Butylene Glycol | 40.00 | 40.00 | 20.00 | 65.00 |
| Vanillyl Butyl Ether | | | | 5.00 |
| PEG-8 | | | 20.00 | |
| Sodium Silicoaluminate | 40.00 | | 20.00 | |
| Calcium Chloride (Anhydrous) | | 40.00 | 20.00 | |
| Mineral Oil | 20.00 | | | 10.00 |
| Dimethicone | | 20.00 | 10.00 | 10.00 |
| Anhydrous Sodium Cocoyl Isethionate | | | 10.00 | |
| Anhydrous Sodium Lauryl Sulfate | | | | 10.00 |

The formulations are prepared by mixing at 70°C. Each is blended with zea mays (destructurized) starch and fed into an extruder at a relative ratio of 1:10. Sheets of foamed starch impregnated with the sample are obtained from the extrusion. These sheets are then sprayed with fragrance oil at a 0.5% loading by weight of the total composition.

### EXAMPLE 18

A destructurized starch is extruded in the form of pellets averaging 10 mm in diameter. The body wash liquid formula as described in the Table below is sprayed at 10% by weight of final composition onto the pellets.

**BODY WASH PELLETS**

| INGREDIENT | WEIGHT % |
|---|---|
| Sodium Lauryl Sulphate | 56.7 |
| Sodium Cocoyl Isethionate | 10.0 |
| Glycerin | 30.0 |
| PEG-7 Glycerol Cocoate | 2.0 |
| Germall 115 | 0.2 |
| Fragrance | 0.5 |
| Vitamin C Acetate | 0.2 |
| Vitamin E Acetate | 0.2 |
| Vitamin A Palmitate | 0.1 |
| Retinol | 0.1 |

### EXAMPLE 19

A hair color concentrate composition is herein illustrated. Addition of water releases the colorant when ready for use and application onto the hair. The composition is prepared by first extruding a sheet of destructurized starch and from this forming pellets with average spherical diameter of about 2 mm. A hair color concentrate cosmetic agent with fragrance is sprayed onto the destructurized starch to form the final cosmetic hair color composition. Components of the cosmetic agent/fragrance liquid sprayed composition are reported in the Table below.

**HAIR COLOR CONCENTRATE**

| INGREDIENT | WEIGHT % |
|---|---|
| Sodium Lauryl Sulphate | 40 |
| Ethanolamine | 10 |
| Colorant* | 12 |
| Glycerin | 30 |
| Fragrance | 4 |
| Sodium Sulphite | 3 |
| Ascorbic Acid | 1 |

| | |
|---|---|
| * Colorant may contain one or more of the following: resorcinol, p-phenylenediamine, m-aminophenol, p-aminophenol, 2-methylresorcinol, 1-napthol, 2-methyl-5-hydroxyethylaminophenol, 4-amino-m-cresol, phenol-methyl-pyrozolone, p-amino-o-cresol, N,N-bis(2-hydroxyethyl)-p-phenylinediamine sulphate and combinations thereof. | |

### EXAMPLE 20

A depilatory solid cosmetic can be prepared by spraying a depilatory concentrate/fragrance in an amount of 20% by weight of the final composition onto a sheet of destructurized starch. The product represented by the final composition can with a small amount of water be activated by a user when ready for the removal of hair. The depilatory concentrate with active cosmetic agent and fragrance has a formula outlined in the Table below.

**DEPILATORY CONCENTRATE**

| INGREDIENT | WEIGHT % |
|---|---|
| PEG-6 Caprylic/Capric Glycerides | 70 |
| Thiobromate | 15 |
| Glycerin | 5 |
| Sodium Cocoyl Glutamate | 5 |
| Fragrance | 5 |

### EXAMPLE 21

This Example evaluates the benefits of utilizing a destructurized starch as a carrier for fragrance.

A pellatized destructurized cornstarch was utilized for the present experiment. These pellets were purchased from Uline Shipping Supplies Corporation, model no. S-1564.

The pellets were then placed in a GE 450 watt, 12 speed blender 2 cups at a time. The pellets were then blended on a "high" setting for 10 seconds resulting in particles between less than 0.1 mm and about 8 mm in diameter. The chopped starch was then poured into a 400 ml beaker up to the 200 ml mark and 0.5 grams of fragrance was sprayed onto the starch using a spray bottle. The starch and fragrance were then mixed together using a spatula for about 20 seconds. The fragranced and mixed starch was then poured into a 4 oz. (120 ml) jar until it was % full and was then capped. The rest of the fragrances starch was discarded.

Thereafter a 400 ml beaker was filled with 200 ml of unmodified cornstarch, Pure-Dent B700, lot #SO203106 from Grain Processing. In the same way, 0.5 grams of fragrance was sprayed onto the starch and it was mixed in the beaker with a spatula for 20 seconds. The starch was then poured into a 4 oz. (120 ml) jar until it was ¾ full. The jar was then capped. The rest of the fragranced starch was then discarded.

The next morning, the caps were removed, the jars were left to sit for one minute with the cap off, and panel testing started. A panelist was given both jars, one filled with chopped, fragranced destructurized starch and the other filled with fragranced unmodified starch and asked, "Which one smells like it expresses fragrance more strongly?" Panelists were allowed to smell each sample as long as they wished. Panelists were also allowed to switch back and forth and smell each sample as often as they wished.

All five of the panelists identified the destructurized starch sample as a clear winner for expressing fragrance over a prolonged period of time.

### EXAMPLE 22

A concentrated skin lotion containing solid of the formula described below was prepared according to the method of Example 1.

**SKIN LOTION SOLID**

| INGREDIENT | WEIGHT % |
|---|---|
| Destructurized Starch | 79.00 |
| Stearic Acid | 5.60 |
| Glycerol Monostearate/Stearamide AMP | 2.00 |
| Glycerolmonostearate | 1.20 |
| Cetyl Alcohol | 0.6 |
| Sunflower Seed Oil | 0.52 |
| Potassium Hydroxide (45% active) | 0.80 |
| Glycerin | 9.28 |
| Fragrance | 1.00 |

## Claims

1. A solid cosmetic composition comprising:
(i) a water dissolvable solid carrier comprising a destructurized starch;
(ii) at least one cosmetic agent incorporated into the solid carrier in an amount sufficient to provide a cosmetic benefit; and
(iii) a fragrance deposited as a post-dose onto the solid carrier that comprises the destructurized starch and the at least one cosmetic agent.

2. The composition according to claim 1 wherein the destructurized starch is a corn starch having an amylose content of at least 45% by weight.

3. The composition according to claim 1 or claim 2 wherein the composition is in a form selected from the group consisting of tablets, pellets, beads and sheets.

4. The composition according to any one of the preceding claims wherein the cosmetic agent is selected from the group consisting of surfactants, emollients, humectants, conditioners, sunscreens, anti-aging actives and mixtures thereof.

5. The composition according to any one of the preceding claims which is in a honeycomb shape.

6. The composition according to claim 5 wherein the honeycomb is formed from a plurality of sections, each of the sections defined by a fracturable perimeter allowing separation of the section from the honeycomb.

7. The composition according to any one of the preceding claims wherein the cosmetic agent is present in an amount from 0.001 to 50% by weight of the composition.

8. The composition according to any one of the preceding claims wherein the fragrance is present in an amount from 0.001 to 10% by weight of the composition.

9. The composition according to any one of the preceding claims wherein the destructurized starch is present in an amount from 10 to 100% by weight of the solid carrier.

10. A foamed solid cosmetic composition comprising:
(i) a water dissolvable foamed solid carrier comprising a destructurized starch;
(ii) at least one cosmetic agent incorporated into the foamed solid carrier in an amount sufficient to provide a cosmetic benefit; and
(iii) a fragrance deposited as a post-dose onto the solid carrier that comprises the destructurized starch and the at least one cosmetic agent.

## Patentansprüche

1. Feste kosmetische Zusammensetzung, umfassend:
(i) einen in Wasser lösbaren festen Träger, umfassend eine destrukturierte Stärke;
(ii) mindestens ein kosmetisches Mittel, das in den festen Träger in einer ausreichenden Menge eingearbeitet ist, um einen kosmetischen Vorteil bereitzustellen; und
(iii) einen Duftstoff, der als eine Nachdosierung auf den festen Träger, der die destrukturierte Stärke und das mindestens eine kosmetisch verträgliche Mittel umfasst, abgeschieden ist.

2. Zusammensetzung nach Anspruch 1, worin die destrukturierte Stärke eine Maisstärke mit einem Amylosegehalt von mindestens 45 Gew.-% ist.

3. Zusammensetzung nach Anspruch 1 oder Anspruch 2, worin die Zusammensetzung in einer Form, ausgewählt aus der Gruppe, bestehend aus Tabletten, Pellets, Perlen und Tüchern, vorliegt.

4. Zusammensetzung nach einem der vorangehenden Ansprüche, worin das kosmetische Mittel, aus der Gruppe, bestehend aus Tensiden, Erweichungsmitteln, Feuchthaltemitteln, Konditionierungsmitteln, Sonnenschutzmitteln, Anti-alterungswirkstoffen und Gemischen davon, ausgewählt ist.

5. Zusammensetzung nach einem der vorangehenden Ansprüche, die in einer Wabenform vorliegt.

6. Zusammensetzung nach Anspruch 5, wobei die Wabe aus einer Vielzahl von Abschnitten gebildet wird, wobei jeder der Abschnitte durch eine Bruchumfangslinie, die die Trennung des Abschnitts von der Wabe erlaubt, definiert ist.

7. Zusammensetzung nach einem der vorangehenden Ansprüche, wobei das kosmetische Mittel in einer Menge von etwa 0,001 bis etwa 50 Gew.-% der Zusammensetzung vorliegt.

8. Zusammensetzung nach einem der vorangehenden Ansprüche, wobei der Duftstoff in einer Menge von etwa 0,001 bis etwa 10 Gew.-% der Zusammensetzung vorliegt.

9. Zusammensetzung nach einem der vorangehenden Ansprüche, wobei die destrukturierte Stärke in einer Menge von etwa 10 bis etwa 100 Gew.-% der Zusammensetzung vorliegt.

10. Geschäumte feste kosmetische Zusammensetzung, umfassend:
(i) einen in Wasser lösbaren geschäumten festen Träger, umfassend eine destrukturierte Stärke;
(ii) mindestens ein kosmetisches Mittel, das in den geschäumten festen Träger in einer ausreichenden Menge eingearbeitet ist, um einen kosmetischen Vorteil bereitzustellen; und
(iii) einen Duftstoff, der als eine Nachdosierung auf dem festen Träger, der die destrukturierte Stärke und das mindestens eine kosmetisch verträgliche Mittel umfasst, abgeschieden ist.

## Revendications

1. Composition cosmétique solide comprenant :
(i) un support solide hydrosoluble comprenant un amidon déstructuré ;
(ii) au moins un agent cosmétique incorporé dans le support solide en une quantité suffisante pour fournir un bénéfice cosmétique ; et
(iii) un parfum déposé sous la forme d'une post-dose sur le support solide qui comprend l'amidon déstructuré et le au moins un agent cosmétique.

2. Composition selon la revendication 1, dans laquelle l'amidon déstructuré est un amidon de mais possédant une teneur en amylose d'au moins 45 % en poids.

3. Composition selon la revendication 1 ou la revendication 2, dans laquelle la composition est sous une forme choisie dans le groupe constitué de pastilles, de granulés, de perles et de feuilles.

4. Composition selon l'une quelconque des revendications précédentes, dans laquelle l'agent cosmétique est choisi dans le groupe constitué de tensioactifs, d'émollients, d'humectants, de conditionneurs, d'écrans solaires, actifs antivieillissement et de leurs mélanges.

5. Composition selon l'une quelconque des revendications précédentes, qui est sous la forme d'un nid d'abeilles.

6. Composition selon la revendication 5, dans laquelle le nid d'abeilles est formé à partir d'une pluralité de sections, chacune des sections étant définie par un périmètre fracturable permettant la séparation de la section du nid d'abeille.

7. Composition selon l'une quelconque des revendications précédentes, dans laquelle l'agent cosmétique est présent en une quantité de 0,001 à 50 % en poids de la composition.

8. Composition selon l'une quelconque des revendications précédentes, dans laquelle le parfum est présent en une quantité de 0,001 à 10 % en poids de la composition.

9. Composition selon l'une quelconque des revendications précédentes, dans laquelle l'amidon déstructuré est présent en une quantité de 10 à 100 % en poids du support solide.

10. Composition cosmétique solide expansée comprenant :
(i) un support solide expansé hydrosoluble comprenant un amidon déstructuré ;
(ii) au moins un agent cosmétique incorporé dans le support solide expansé en une quantité suffisante pour fournir un bénéfice cosmétique ; et
(iii) un parfum déposé sous la forme d'une post-dose sur le support solide qui comprend l'amidon déstructuré et le au moins un agent cosmétique.
